# FASCICULE DE BREVET EUROPEEN

(11) **EP 0 845 964 B1**
(45) Date de publication et mention de la délivrance du brevet: **26.01.2005**
(21) Numéro de dépôt: 96929358.8
(22) Date de dépôt: 22.08.1996
(51) Int. Cl.: A61F 2/32, A61F 2/38, A61B 17/17

(54) **ELEMENTS DE PROTHESE D'ARTICULATION ET PROCEDE DE FABRICATION DE CEUX-CI**
ELEMENTE FÜR GELENKPROTHESE UND VERFAHREN ZU DEREN HERSTELLUNG
JOINT PROSTHESIS MEMBERS AND METHOD FOR MAKING SAME

(30) Priorité: 23.08.1995 FR 9510004
(43) Date de publication de la demande: 10.06.1998
(73) Titulaire: Chibrac, Jean, 33600 Pessac (FR); Gemon, Jean-Pierre, 33000 Bordeaux (FR)
(72) Inventeur: CHIBRAC, Jean, F-33600 Pessac (FR); GEMON, Jean-Pierre, F-33000 Bordeaux (FR); LE REBELLER, Alain, F-33000 Bordeaux (FR)
(74) Mandataire: Dronne, Guy
(86) Numéro de dépôt international: PCT/FR1996/001306
(87) Numéro de publication internationale: WO 1997/007753

(56) Documents cités:
- EP-A- 0 317 972
- WO-A-89/07910
- CH-A- 449 173
- DE-A- 2 933 174
- DE-A- 3 516 743
- FR-A- 2 630 640
- FR-A- 2 686 503
- US-A- 3 547 115

## Description

La présente invention a pour objet des éléments de prothèse d'articulation, un ensemble de mise en place des éléments de prothèse d'articulation et un procédé de fabrication desdits éléments de prothèse d'articulation.

De nombreuses maladies telles que l'arthrose ou les rhumatismes ou encore certains types d'accidents conduisent à des dégradations des différentes articulations et notamment des grosses articulations telles que la hanche, le genou, l'épaule etc. Ces problèmes ou accidents se traduisent plus particulièrement par la destruction des cartilages constituant la surface d'articulation. Cette dégradation, notamment des cartilages, peut conduire à un handicap sérieux pour le patient qui en est victime et même à une incapacité lourde.

Dans ce domaine, on connaît bien les éléments de prothèse utilisés pour l'articulation du genou ou de la hanche.

Les éléments de prothèse utilisés dans ce cas nécessitent selon les techniques actuelles une résection importante des surfaces articulaires, cette résection se traduisant soit par la réalisation d'une surface plane à l'extrémité de l'os concerné, soit par la réalisation de plusieurs surfaces planes angulées les unes par rapport aux autres constituant une approximation de la surface réelle d'articulation.

Ce type d'éléments de prothèse nécessite pour sa mise en place un e résection importante de l'os. Cette résection nécessite à son tour la mise en oeuvre d'un matériel chirurgical lourd pour définir précisément le plan ou les plans de la surface de résection à réaliser. En outre, la surface de résection étant plane ou constituée par plusieurs portions de plan, il est nécessaire de prévoir un ancrage très important en profondeur de l'élément de prothèse dans la partie de l'os associé à la surface articulaire pour assurer la solidarisation de la prothèse. Ces éléments d'ancrage tels que vis, broches, plots, etc.. nécessitent le forage de trous d'ancrage importants dans l'os à traiter. Ce forage ou perçage important nécessite à son tour la mise en oeuvre d'un matériel chirurgical lourd. En outre, l'importance du forage ou du perçage dans l'os peut entraîner des conséquences dommageables pour la partie de l'os qui a subi cette perforation, notamment en ce qui concerne sa résistance mécanique lorsque des contraintes sont appliquées aux os constituant cette articulation lors de mouvements effectués puisque, par sa longueur, l'élément d'ancrage reporte l'effort en une zone de l'os qui n'est pas prévue pour supporter cet effort.

De plus, dans le cas d'une résection importante sensiblement plane, l'appui de la prothèse sur la surface de résection est non satisfaisant car cette surface est essentiellement constituée par de l'os spongieux, l'appui sur la périphérie corticale de l'os étant insuffisant quant aux reports d'efforts.

En outre, les éléments de prothèse connus n'offrent pas aux praticiens un choix suffisant de gamme pour répondre aux degrés de liberté articulaire choisis. Enfin, les moyens d'ancrage de ces prothèses restent encore un élément d'interrogation en ce qui concerne leur solidité, leur stabilité et leur usure.

Un objet de la présente invention est de fournir des éléments de prothèse pour les différentes articulations du corps humain qui ne nécessitent qu'une résection de la surface articulaire limitée tout en présentant un ancrage très efficace sur la partie de l'os concernée et qui ne nécessite qu'un matériel chirurgical plus simple pour leur mise en place. Un autre objet de l'invention est de fournir un tel élément de prothèse articulaire qui, dans le cas ou l'ensemble des ligaments de l'articulation reste intact, permet de redonner au patient l'ensemble des degrés de liberté de l'articulation saine.

Un autre objet de l'invention est de fournir un élément de prothèse qui favorise la transmission des efforts appliqués à la prothèse vers l'os.

Pour atteindre ce but, selon l'invention, l'élément de prothèse d'articulation est conforme aux caracteristiques techniques de la revendication 1.

On comprend que, grâce notamment à la forme de la surface de contact sur la partie de la surface articulaire ayant subi la résection et grâce à la présence de la surface périphérique de l'élément de prothèse qui coopère avec le rebord en saillie de la résection, on obtient déjà une bonne solidarisation de l'élément de prothèse sur la surface articulaire de l'os après la résection. On comprend qu'ainsi, il suffit de prévoir un élément d'ancrage relativement peu important et ne pénétrant dans l'os que sur une longueur réduite ou une couche d'un matériau de collage ou de scellement pour immobiliser totalement et parfaitement l'élément de prothèse sur l'extrémité de l'os.

Un autre objet de l'invention est de fournir un ensemble de mise en place de prothèses d'articulation du type défini precécemment sur une surface articulaire d'un des os de ladite articulation caractérisé en ce qu'il comprend:
- un ensemble de prothèses semblables se distinguant par certaines côtes, chaque prothèse comprenant une face de contact pour coopérer avec une prothèse d'un autre type fixée sur l'autre os de ladite articulation, une face de fixation destinée à être fixée sur ladite surface articulaire de l'os après résection convenable de celui-ci, ladite face de fixation étant munie d'au moins un élément d'ancrage faisant saillie dans ladite face et une surface périphérique reliant lesdites faces de contact et de fixation, chaque élément de prothèse ayant une épaisseur sensiblement constante au moins dans sa partie centrale entre lesdites deux faces;
- un premier ensemble de moyens formant gabarits de contour de résection, chaque gabarit étant associé à une prothèse de l'ensemble, chaque gabarit de contour comportant des moyens de traçage du contour de la résection à réaliser, ledit contour correspondant à la périphérie de ladite prothèse;
- un ensemble de moyens formant gabarits de trous d'ancrage, chaque moyen formant gabarit étant associé à une des prothèses de l'ensemble, chaque gabarit de trou d'ancrage comportant des moyens de positionnement par rapport au contour de résection et des moyens de traçage de l'emplacement du ou des trous d'ancrage associés aux éléments d'ancrage;
- un premier ensemble d'instruments de fraisage pour réaliser ladite résection à l'intérieur dudit contour et contrôler les paramètres de fraisage;
- un deuxième ensemble d'instruments de forage pour réaliser lesdits trous d'ancrage et contrôler les paramètres de forage; et
- des moyens d'aide au guidage du déplacement desdits instruments de telle manière que les instruments de fraisage soient déplacés au moins selon ledit contour de telle manière que leur axe reste sensiblement parallèle à une direction fixe, ladite direction étant liée auxdits gabarits et que les instruments de forage soient maintenus avec le même axe de perçage.

On comprend que, grâce à l'utilisation d'une part de gabarits matériels ou optiques permettant le traçage du contour de la résection et le positionnement du ou des trous d'ancrage de l'élément de prothèse et grâce à l'utilisation de fraisage pour réaliser la résection, on obtient effectivement une résection qui permet l'utilisation d'un élément de prothèse dont la surface périphérique coopère mécaniquement avec le rebord en saillie de la résection obtenue.

Plus généralement, on comprend que l'invention, dans ses différents aspects, permet d'utiliser des éléments de prothèse pour les différentes articulations dont la forme coïncide avec la résection réalisée par le chirurgien lors de la mise en place des éléments de prothèse. On comprend également que, grâce à l'épaisseur sensiblement constante de ces éléments de prothèse et grâce à la surface périphérique de ces éléments de prothèse qui vient en appui sur le rebord de la résection, on a une immobilisation de l'élément de prothèse qui ne nécessite pas la mise en oeuvre d'un ancrage important de l'élément de prothèse sur l'os considéré et, en particulier, qui ne pénètre plus sur une longueur importante dans l'os.

Un autre objet de l'invention est de fournir un procédé de fabrication de l'élément de prothèse d'articulation défini précédemment caractérisé en ce qu'il comprend les étapes suivantes:
- on part d'un os ayant la forme exacte de la surface articulaire d'un os naturel pour lequel on veut fabriquer la prothèse;
- on fraise une partie de l'extrémité de l'os selon ladite surface articulaire en maintenant une direction constante de fraisage de manière à définir une zone centrale fraisée et une surface périphérique, la profondeur de fraisage étant sensiblement constante dans ladite zone centrale, ladite surface périphérique définissant au moins en partie un rebord en saillie par rapport à ladite zone centrale selon la direction de fraisage;
- on réalise un moule dont l'empreinte est limitée d'une part par une surface analogue à la surface articulaire de l'os avant fraisage et d'autre part par la zone centrale fraisée et ladite surface périphérique résultant du fraisage:

- on introduit dans ledit moule un matériau de moulage pour que celui-ci prenne la forme de l'empreinte du moule;
- on démoule la pièce ainsi obtenue par quoi on obtient un prototype dudit élément de prothèse; et
- on réalise ledit élément de prothèse à partir dudit prototype.

Grâce au procédé de fabrication des éléments de prothèse, il est possible de donner à la face de contact de l'élément de prothèse une forme identique ou quasi-identique à celle de la surface articulaire anatomique avant la résection. Cette disposition est particulièrement intéressante lorsque le système de ligaments de l'articulation demeure intact chez le patient à équiper de la prothèse. En revanche, dans le cas où le système de ligaments a été affecté et où les degrés de liberté anatomique doivent être limités en conséquence, il est possible de donner à la surface de contact de l'élément de prothèse une forme modifiée présentant une congruence plus importante avec la surface de contact de l'élément de prothèse associé afin de limiter les possibilités de mouvement relatif des os formant l'articulation.

Selon un premier mode de mise en oeuvre, les moyens de fixation sont des ergots de forme générale conique qui pénètrent dans des trous d'ancrage de même forme.

Selon un deuxième mode de mise en oeuvre, les moyens de fixation comprennent un élément d'ancrage qui a une forme en queue d'aronde raccordée à la partie centrale de la surface de fixation. Dans ce cas, le trou ou orifice d'ancrage présente la forme conjuguée de l'élément d'ancrage. Pour permettre la mise en place de l'élément de prothèse, on comprend que le trou d'ancrage doit déboucher à une extrémité, alors qu'il est obturé à son autre extrémité par l'os non réséqué. Dans ce cas, l'ancrage est avantageusement complété par une vis ou broche engagée dans l'extrémité libre de l'élément d'ancrage afin d'immobiliser en translation l'élément de prothèse.

Selon un troisième mode de mise en oeuvre, les moyens de fixation comprennent une couche de matériau de scellement ou de collage.

D'autres caractéristiques et avantages de l'invention apparaîtront mieux à la lecture de la description qui suit de plusieurs modes de réalisation de l'invention donnés à titre d'exemples non limitatifs. La description se réfère aux figures annexées sur lesquelles:
- La figure 1 est une perspective de la résection réalisée sur l'extrémité supérieure du tibia;
- Les figures 1a et 1b sont des vues en perspective des éléments de prothèse à mettre en place sur l'extrémité supérieure du tibia;
- La figure 1c est une vue en coupe de l'élément de prothèse de la figure la mis en place;
- La figure 2a est une vue simplifiée en coupe d'un premier type d'élément de prothèse;
- La figure 2b est une vue en coupe d'un deuxième type d'élément de prothèse;
- Les figures 2c et 2d sont des vues en coupe de troisième et quatrième types d'éléments de prothèse;
- Les figures 2e à 2h illustrent une variante de réalisation des figures 2a à 2d dans laquelle l'élément de prothèse est dépourvu d'élément d'ancrage.
- La figure 3 est une vue en élévation illustrant la première étape du procédé de fabrication d'un élément de prothèse;
- La figure 4 est une vue de dessus de la surface articulaire d'un os montrant la définition de la résection;
- Les figures 5a, 5b et 5c illustrent deux types de fraise utilisables pour la fabrication d'un élément de prothèse;
- La figure 6 est une vue en coupe verticale d'un moule utilisable pour la fabrication d'un prototype d'élément de prothèse.

Avant de décrire en détail la forme des éléments de prothèse, on va rappeler le principe général de la présente invention. Les éléments de prothèse sont implantés dans des résections réalisées au préalable par le chirurgien sur les surfaces articulaires. Grâce à l'ensemble des instruments, qui seront décrits ultérieurement, mis à la disposition des chirurgiens pour réaliser ces résections et grâce au mode de fabrication des éléments de prothèse, le procédé de fabrication étant décrit ultérieurement, il est possible d'obtenir une relation géométrique étroite entre la forme de la résection réalisée dans la surface articulaire et la forme de l'élément de prothèse à mettre en place. On obtient ainsi une liaison mécanique de grande qualité entre l'élément de prothèse et l'os correspondant.

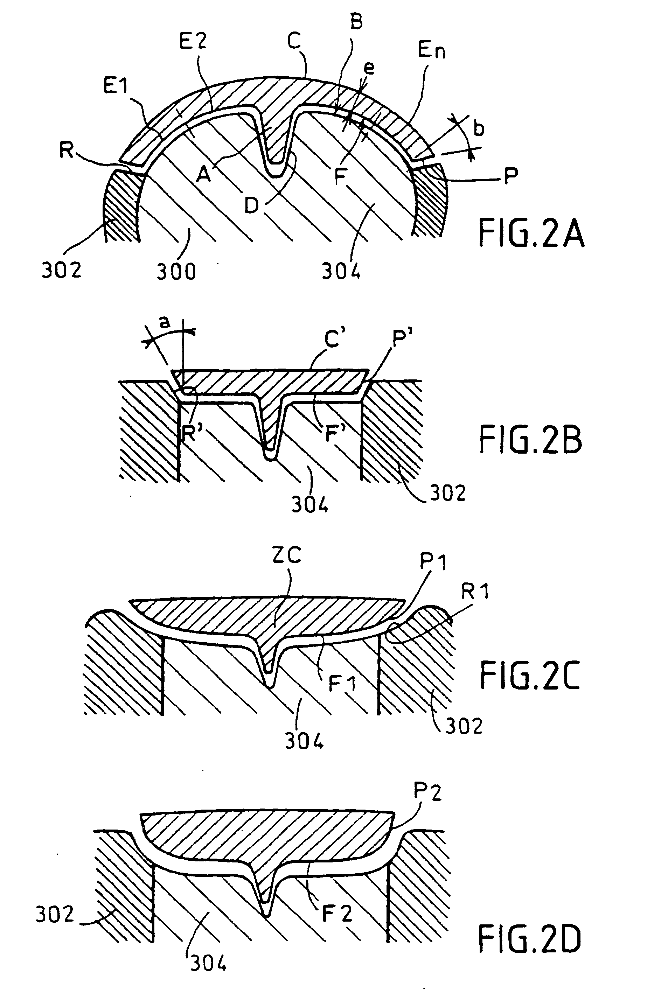

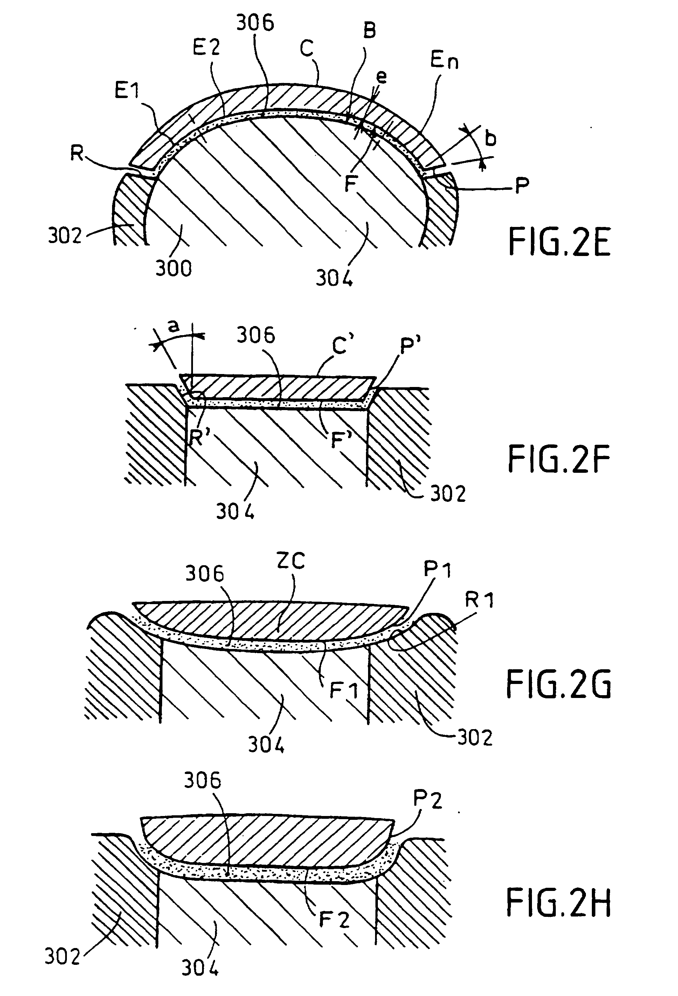

Comme le montre la figure 2a, chaque élément de prothèse est limité par une surface de contact C, par une surface de fixation F et par une surface périphérique P qui raccorde les deux surfaces C et F. En outre, la surface de fixation F est munie éventuellement d'au moins un élément d'ancrage A qui fait saillie hors de la face de fixation F en faisant partie intégrante de la prothèse.

Si maintenant on considère la résection réalisée par le chirurgien dans la surface articulaire pour permettre la mise en place de l'élément de prothèse, celle-ci comprend une surface d'appui B, un rebord périphérique en saillie par rapport à la surface d'appui B qui est référencé R et éventuellement au moins un orifice d'ancrage D. Cette organisation générale de l'élément de prothèse et de la résection correspondante se retrouve quelle que soit la surface articulaire pour laquelle est mis en place l'élément de prothèse.

Comme le montre la figure 2a, l'os 300 dans la zone de la résection comporte une portion corticale périphérique 302 d'épaisseur réduite et présentant une grande résistance mécanique et une portion centrale 304 constituée par de l'os spongieux dont on a mis en évidence qu'il présentait une résistance mécanique bien moindre. La résection est réalisée de telle manière que le rebord R soit intégralement constitué par la portion carticale 302 de l'os.

Comme on l'expliquera ultérieurement grâce aux instruments mis à la disposition du chirurgien pour effectuer la résection et grâce à la technique particulière de fabrication de l'élément de prothèse, il est possible d'obtenir une concordance. géométrique sensiblement parfaite entre la surface de fixation F et la surface d'appui B de la résection. L'élément de prothèse est ainsi encastré dans la résection. De même, il est possible d'obtenir un contact sensiblement parfait après mise en place de la surface périphérique P de la prothèse et du rebord périphérique R de la résection. Enfin, il y a également coïncidence entre l'élément d'ancrage A lorsqu'il existe et le trou d'ancrage D.

Dans sa partie courante, l'élément de prothèse présente une épaisseur e qui est sensiblement constante. Les essais effectués ont montré que, compte-tenu du matériau utilisé pour réaliser les éléments de prothèse, il est nécessaire que l'épaisseur e sensiblement constante de l'élément de prothèse soit supérieure à 6 mm. En effet, ces essais ont montré qu'en dessous de cette valeur, l'élément de prothèse subissait un phénomène de fluage très préjudiciable à la bonne résistance mécanique de la prothèse dans le temps. La surface d'appui B de la résection présente bien sûr une forme correspondant à celle de la surface de fixation F. On comprend que grâce d'une part à la présence de la surface périphérique P de l'élément de prothèse qui est en appui sur le rebord en saillie R de la résection et de la forme particulière de la surface de fixation, l'élément de prothèse mis en place est déjà liée mécaniquement à l'os. On comprend qu'ainsi, il est possible que l'élément d'ancrage A, lorsqu'il existe, soit simplement constitué par un ergot qui pénètre dans l'orifice D de la résection. Il n'est plus nécessaire de prévoir un système de broches, de vis, plots ou similaire. En ce qui concerne la surface de contact C qui est destinée à coopérer avec la surface de contact de l'élément de prothèse conjugué de l'articulation, elle est réalisée de préférence de telle manière qu'elle se conforme exactement à la forme anatomique de la surface articulaire. Après mise en place de l'élément de prothèse dans la résection, on reconstitue ainsi exactement la surface articulaire anatomique initiale. Il faut ajouter que la résection est réalisée de telle manière que, lorsque cela est possible, elle n'affecte pas les points d'attache des ligaments et des muscles, ni de la capsule articulaire et les surfaces articulaires environnantes.

En première approximation, la surface périphérique P a la forme d'un tronc de cône non circulaire.

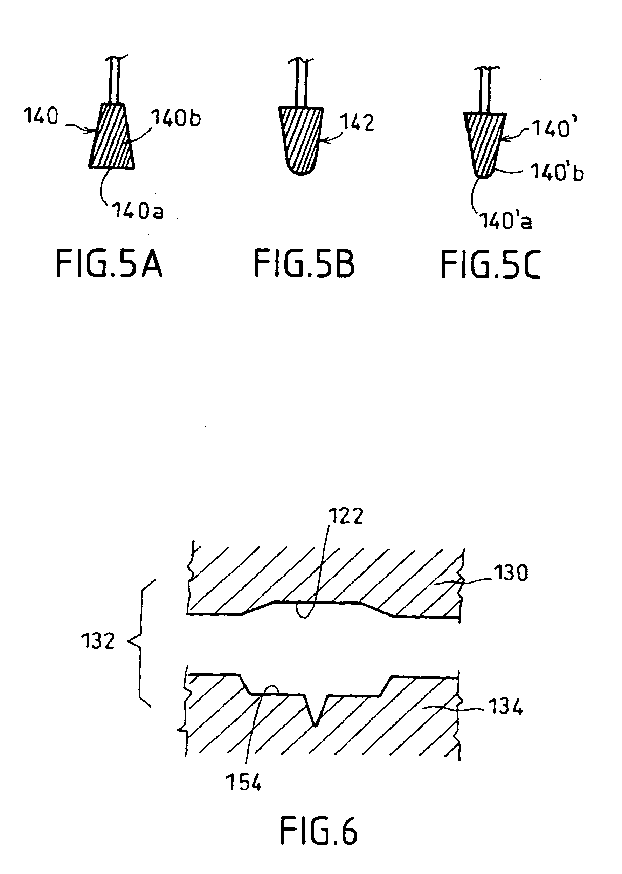

Dans le cas de l'élément de prothèse représenté sur la figure 4B, la surface de contact C' et la surface de fixation F' sont sensiblement planes et parallèles entre elles dans leurs parties courantes, l'épaisseur étant sensiblement constante. La surface périphérique P' est en première approximation un tronc de cône dont le demi-angle au sommet est de l'ordre de 5° à 45°. Le rebord périphérique R' présente une forme conjuguée de la forme de la surface périphérique P'. Pour ce type d'éléments de prothèse, la résection effectuée est assimilable à un lamage réalisé par le chirurgien dans la surface articulaire.

Le sommet du cône non circulaire sur lequel est disposée la surface périphérique P' peut être disposé du côté de la surface de fraction F comme sur la figure 4b. Pour certaines surfaces articulaires, notamment dans le cas des vertèbres, le sommet du cône peut être disposé du côté de la surface de contact C.

Dans le cas des figures 2a et 2b, la surface périphérique de fixation P ou P' est nettement distincte de la surface de fixation F ou F'.

Dans le cas de l'élément de prothèse de la figure 2c, la surface périphérique P1 est une solution de continuité de la surface de fixation F1, ces deux surfaces se confondant sensiblement. Il va également de soi que dans ce cas, seule la partie centrale ZC de l'élément de prothèse présente une épaisseur sensiblement constante. Cependant dans ce cas encore, l'élément de prothèse est encastré dans la résection et la surface périphérique P1 coopère avec le rebord R1 de la résection pour participer à la fixation de la prothèse.

Dans le cas de la figure 2d, la surface périphérique P2 se distingue nettement de la surface de fixation F2, mais la section droite par un plan de cette surface périphérique n'est plus un segment rectiligne, comme dans le cas des figures mais une portion de courbe. La surface périphérique est alors une surface gauche qui n'est plus assimilable à un tronc de cône 2a et 2b.

Quel que soit le type d'éléments de prothèse considérés, on comprend que la surface périphérique P P' joue un rôle déterminant en coopérant avec le rebord en saillie de la résection. En effet, ce contact permet la répartition optimale de la transmission des efforts entre l'os et la prothèse, ce qui permet, comme on l'a déjà expliqué, d'éviter un ancrage par vis, broches ou plats de dimensions importantes ou analogue de la prothèse sur l'os. Il est même possible de supprimer tout élément d'ancrage et de le remplacer par une couche de matériau de collage ou de scellement interposée entre la face de fixation de la prothèse et le fond de la résection. Dans ce cas, de préférence, la face de fixation présente une certaine rugosité pour favoriser l'adhérence du matériau. C'est ce qui est représenté sur les figures 2h à 2k. Elles correspondent aux figures 2a à 2d mais l'élément d'ancrage A est supprimé. On a référencé 306 la couche de matériau de collage ou de scellement dont l'épaisseur a été exagérée.

Il faut ajouter que dans la suite de la description, on décrit plus particulièrement le cas où les moyens de fixation de la prothèse sur le fond de la résection consistent en au moins un élément d'ancrage. Cependant, on ne sortirait pas de l'invention si l'élément d'ancrage était remplacé ou complété par une couche de matériau de collage ou de scellement.

Il est très important de souligner que l'angulation de la partie périphérique P ou P' de l'élément de prothèse qui est au contact du rebord R de la résection permet d'augmenter la surface de contact entre ces deux parties. En outre, cette angulation permet de modifier la direction de transmission des efforts verticaux auxquels est soumise la prothèse vers l'os. Plus précisément, cette transmission se fait orthogonalement à la surface de contact. Il y a donc une composante de ces efforts qui tend à appliquer effectivement l'élément de prothèse sur le rebord R de la résection. Cela améliore encore l'effet d'encastrement de la prothèse dans la résection.

De plus, comme le rebord de la résection est constitué par la portion carticale de l'os, les efforts transmis par le contact entre la périphérie de la prothèse et le rebord de la résection le sont à la partie de l'os la plus résistante. Selon la forme de l'élément de prothèse, il est possible de déterminer l'angulation de la périphérie de la prothèse de telle manière qu'au moins 70% des efforts totaux appliqués à la prothèse soient "repris" par la portion corticale de l'os. Le reste des efforts est repris par le contact, de surface relativement importante, entre la face de fixation de la prothèse et la portion d'os spongieux.

La définition de l'angulation de la zone périphérique de la prothèse est un compromis entre l'intérêt qu'il y a à augmenter la surface de contact avec le rebord de la résection correspondant à la zone corticale et le fait qu'il est souhaitable que la plus grande partie de la prothèse ait une épaisseur supérieure à 6 mm afin d'éviter le fluage du matériau. Il va en effet de soi que l'augmentation de la surface de contact entraîne une diminution de la prothèse dans sa zone périphérique.

On comprend que si l'on considère un élément de prothèse complet proprement dit, selon le plan de section considéré, on pourra avoir une configuration similaire à celle de la figure 2a ou bien une configuration similaire à celle de la figure 2b, 2c, ou 2d, ou encore, selon les plans de coupe considérés, une combinaison de ces différentes configurations.

Dans les exemples d'élément de prothèse décrits en liaison avec les figures 2a à 2d, l'élément d'ancrage est constitué par un ou plusieurs ergots de forme générale conique ou tronconique qui fait saillie dans la surface de fixation et le trou d'ancrage a une forme conjuguée qui débouche dans le fond de la résection.

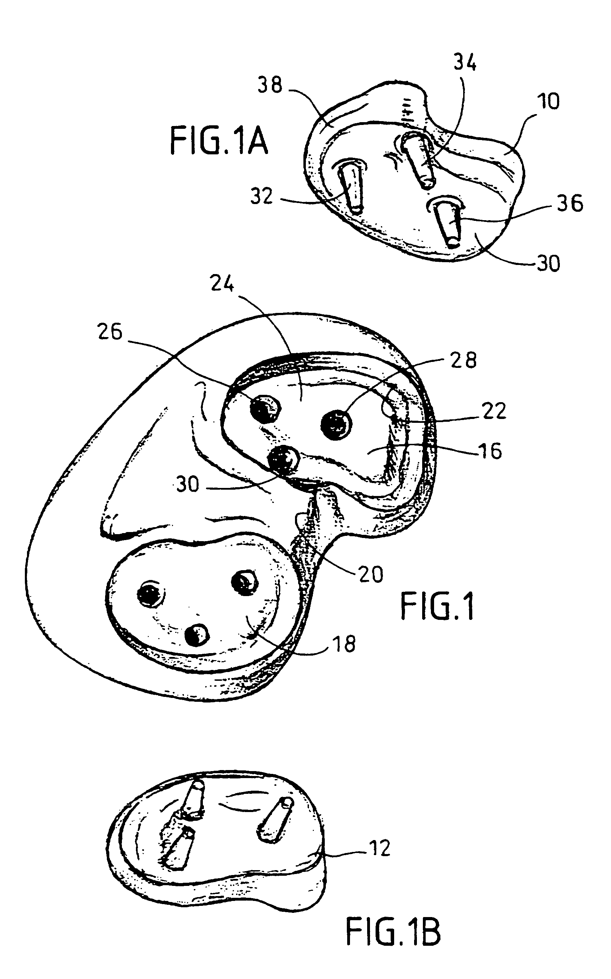

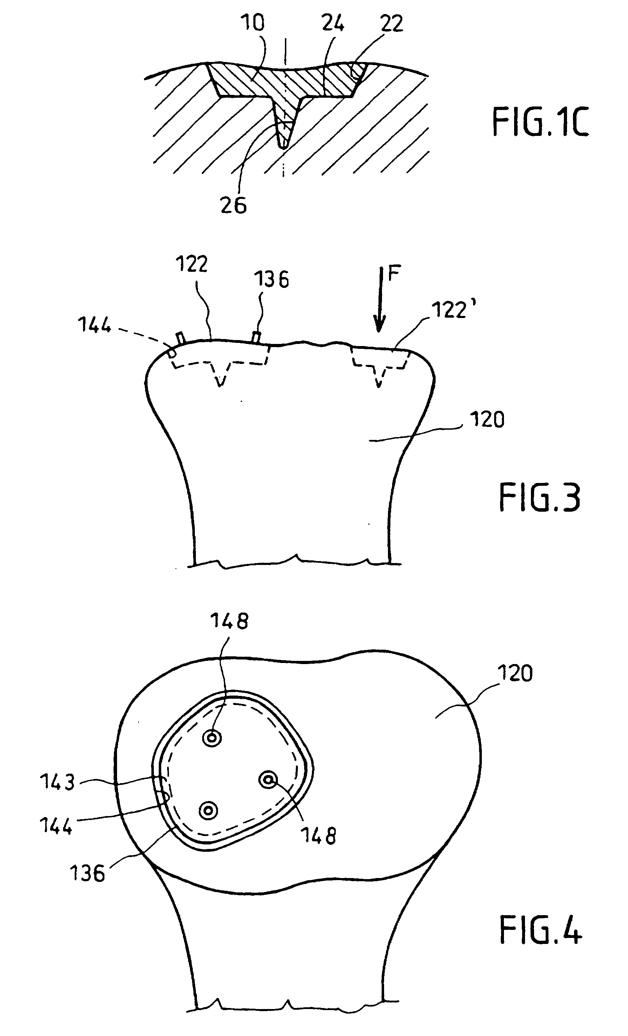

En se référant maintenant aux figures 1 à 1c, on va décrire un exemple concret d'éléments de prothèse correspondant à une articulation particuliere.

Sur les figures 1 à 1c, on a représenté les deux éléments de prothèse 10 et 12, utilisés pour l'extrémité supérieure du tibia. La figure 1 montre les deux résections 16 et 18 qui sont réalisées en conservant les surfaces pré et rétrospinales et donc les insertions LCA et LCD, des ménisques et de la capsule. Chaque résection comporte un rebord d'appui en saillie 22, une surface d'appui 24 et trois trous d'ancrage 26, 28 et 30. Chaque élément de prothèse comporte une surface de fixation 30 qui est sensiblement plane dans laquelle font saillie trois éléments d'ancrage 32, 34 et 36. La surface périphérique 38 a une forme générale de tronc de cône, les différences d'épaisseur dans la zone périphérique étant dues au fait que la surface de contact doit se raccorder tangentiellement à l'os.

Comme le montre mieux la figure 1c, les éléments de prothèse 10 et 12 sont du type à surface de fixation et de contact sensiblement planes.

Comme on l'a déjà indiqué, une des caractéristiques essentielles des éléments de prothèse consiste dans le fait que la fixation mécanique des éléments de prothèse sur la surface articulaire réséquée est essentiellement obtenue par la surface périphérique de fixation de l'élément de prothèse et par sa surface de fixation. Ce résultat est obtenu grâce à la très grande similitude entre ces parties de l'élément de prothèse et le rebord périphérique et la surface d'appui de la résection réalisé par le chirurgien lors de la mise en place de l'élément de prothèse. Ces résultats peuvent être obtenus grâce d'une part aux moyens mis à la disposition du chirurgien pour réaliser la résection et d'autre part au procédé de fabrication des éléments de prothèse.

En se référant maintenant aux figures 3 à 6, on va décrire un procédé de fabrication d'un élément de prothèse conforme à l'invention. Dans un premier temps, illustré par la figure 3, on part d'un os ayant la forme exacte de la surface articulaire d'un os naturel pour laquelle on veut fabriquer la prothèse 120 qui présente la surface articulaire 122 sur laquelle on veut mettre en place l'élément de prothèse. Dans le cas particulier envisagé, il y a deux surfaces articulaires 122 et 122'. On ne décrira que le mode de réalisation de l'élément de prothèse correspondant à la surface articulaire 122. On réalise une reproduction de la forme de la surface anatomique externe de la surface articulaire 122 pour fabriquer une première partie 130 d'un moule 132 dont la face interne définissant une partie de l'empreinte du moule reproduit exactement la forme de la surface articulaire.

Dans un deuxième temps, on va réaliser sur l'os 120 un fraisage correspondant exactement à la résection que le chirurgien effectuera sur la surface articulaire du patient pour la mise en place de l'élément de prothèse. A l'aide d'un gabarit définissant le contour de la résection, ce gabarit étant référencé 136, on définit le contour de la résection. Ce gabarit de traçage du contour peut être matériel ou il peut être optique. Du point de vue anatomique, ce contour correspond sensiblement au contour du cartilage. Après avoir tracé le contour de la résection, à l'aide d'une fraise 140 ou 142 du type illustré sur les figures 5a à 5c, on fraise une gorge suivant le pourtour 36 de la résection avec une profondeur prédéterminé défini par la fraise 140, 140' ou 142. La forme de la fraise est telle que cette gorge 143 définit le rebord en saillie 144 de la résection. A l'aide d'autres instruments de fraisage ou de meulage adaptés, on enlève la partie de la surface articulaire limitée par la gorge 143 qui vient d'être fraisée par une technique telle qu'on enlève en tous points sensiblement la même épaisseur de matière osseuse, ou plus précisément, cartilagineuse. Il est important de préciser que, durant toutes les opérations de fraisage pour réaliser la résection, les différents outils de fraisage sont maintenus avec leur axe parallèle à une direction fixe représentée par la flèche F sur la figure 5. La fraise 140 ou 140' présente une surface de fraisage non seulement vers son extrémité 140a, (140'a) mais également selon sa surface latérale 140, (140'b). On comprend que selon la position du tracé du contour de résection par rapport à la forme de la surface articulaire, on obtiendra effectivement une gorge (fig. 1 ou 4) ou une surface plane ou sensiblement cylindrique. De même, selon les cas, le rebord de la résection sera fraisé par l'extrémité de la fraise ou par sa surface latérale (rebord de la fig. 1, De préférence également l'axe des instruments de perçage ou forage servant à réaliser les trous d'ancrage, lorsque ceux-ci ont une forme générale tronconique, est maintenu parallèle à la direction F de forage. Cependant, on peut prévoir une certaine angulation de l'axe par rapport à cette direction. Ces trous d'ancrage sont de préférence tronconique tout comme l'élément d'ancrage lui-même. Dans un deuxième temps, à l'aide d'un gabarit de forage, on trace la position des trous d'ancrage à réaliser dans la résection, ces trous d'ancrage étant obtenus par un outil de forage ou de fraisage de type convenable. Les trous d'ancrage sont référencés 148 sur la figure 4. On réalise alors par tout moyen convenable un relevé de la forme de la résection ainsi réalisée, c'est-à-dire de sa surface périphérique 150, de sa surface d'appui 152 et des trous d'ancrage 148. A partir de la prise de forme de la résection, on fabrique un deuxième élément de moule 134 dont la face interne qui définit une partie de l'empreinte du moule qui est référencée 154 a la forme exacte de la résection. Il en va bien sûr de même lorsque l'élément d'ancrage est en forme de queue d'aronde. A partir du moule 132, on injecte un matériau de moulage convenable telle qu'une résine ou cire ou autres matériaux, après avoir refermé le moule. Après démoulage, on obtient ainsi un prototype de l'élément de prothèse qui a exactement la forme souhaitée quant à sa face de contact, sa face de fixation et sa surface périphérique.

A partir de ce prototype, on peut, par copie, fabriquer les éléments de prothèse proprement dits avec le matériau bio-compatible convenable tel que du titane, du polyéthylène, ou une combinaison de ces matériraux..

L'invention concerne également un ensemble de mise en place d'éléments de prothèse qui est à la disposition du chirurgien.

Pour chaque élément de prothèse correspondant à une surface articulaire, le chirurgien dispose de plusieurs "tailles" d'éléments de prothèse qui se distinguent les uns des autres seulement par des cotes différentes, la forme des éléments de prothèse étant toujours la même. Avant l'intervention, le chirurgien détermine par tout moyen convenable la taille appropriée.

Pour chaque taille d'élément de prothèse, le chirurgien dispose d'un gabarit pour le traçage du contour de la résection à réaliser et un gabarit de perçage des trous d'ancrage. Ces gabarits peuvent être soit matériels, soit optiques. Dans ce deuxième cas, le gabarit consiste en des moyens de projection sur la surface articulaire de l'image du tracé du contour ou du tracé des trous d'ancrage.

Enfin, le chirurgien dispose d'instruments de fraisage, de meulage et/ou de forage pour réaliser la résection et les trous d'ancrage selon leur forme. Des exemples de fraises sont donnés sur les figures 5a, 5b et 5c. De préférence, chaque fraise ou chaque instrument de forage comporte un repère de profondeur de telle manière que le chirurgien réalise la résection avec la profondeur voulue qui est sensiblement constante.

Comme on l'a déjà indiqué, durant au moins toute l'opération de fraisage du contour de la résection, l'axe de rotation de la fraise doit rester parallèle à une direction donnée qui est définie pour chaque élément de prothèse.

Le chirurgien peut disposer de moyens de guidage matériels ou optiques pour l'aider à maintenir manuellement la fraise dans la direction voulue.

## Revendications

1. Elément de prothèse d'articulation destiné à être fixé sur la surface articulaire d'un os de l'articulation après résection convenable de ladite surface articulaire et à coopérer avec au moins un autre élément de prothèse fixé sur la surface articulaire d'un autre os de l'articulation, ladite résection définissant sur son contour un rebord (22, 98, R, R1) en saillie, ledit élément de prothèse (10, 12, 50, 92) étant limité par une surface de contact (C, C', 66) destinée à coopérer avec l'autre élément de prothèse, ladite surface de contact étant sensiblement identique à la surface articulaire anatomique, une surface de fixation destinée à être fixée sur la résection de la surface articulaire et une surface périphérique (P, P', 38, 74, 76, 102) de raccordement desdites surfaces de contact et de fixation, ladite surface périphérique étant destinée à coopérer avec le rebord en saillie de ladite résection qui correspond à la partie corticale de l'os, ladite surface de fixation étant munie de moyens de fixation (32, 34, 36, 60, 106, A), ledit élément de prothèse étant **caractérisé en ce que** ladite surface de fixation (F') est sensiblement plane, **en ce que** ladite surface de contact (C') est également sensiblement plane et sensiblement parallèle à la surface de fixation, **en ce que** ladite surface de contact est en contact direct avec la surface de contact de l'autre élément de prothèse et **en ce que** lesdits moyens de fixation sont choisis dans le groupe comprenant des matériaux de scellement et au moins un ergot d'ancrage faisant partie intégrante de ladite prothèse, la distance, selon une direction fixe entre lesdites surfaces de contact et de fixation dans sa zone centrale étant sensiblement constante et supérieure à 6 mm, ladite surface périphérique de raccordement allant en s'évasant par rapport à ladite direction fixe depuis la surface de fixation vers la surface de contact en faisant avec ladite direction fixe un angle compris entre 5 et 45 degrés, par quoi ladite surface périphérique est apte à être en appui sur le rebord de ladite résection.

2. Elément de prothèse selon la revendication 1, **caractérisé en ce que** les moyens de fixation comprennent une couche d'un matériau de collage ou de scellement, et **en ce que** la surface de fixation présente une rugosité suffisante pour favoriser l'adhérence dudit matériau.

3. Elément de prothèse selon la revendication 1, **caractérisé en ce que** les moyens de fixation comprennent au moins un élément d'ancrage faisant saillie hors de la surface de fixation et faisant partie intégrante dudit élément de prothèse.

4. Elément de prothèse selon l'une quelconque des revendications 1 à 3, **caractérisé en ce que** ladite surface périphérique (P) a sensiblement la forme d'un tronc de cône à section droite non circulaire.

5. Elément de prothèse selon la revendication 3, **caractérisé en ce que** l'élément d'ancrage (A) comprend au moins un ergot de forme sensiblement tronconique réalisé avec le même matériau que le reste de l'élément de prothèse.

6. Ensemble de mise en place d'un élément de prothèse d'articulation selon l'une quelconque des revendications 1 à 5 sur une surface articulaire d'un des os de ladite articulation, **caractérisé en ce qu'**il comprend :
- un ensemble desdits éléments de prothèse semblables se distinguant par certaines côtes, chaque élément de prothèse comprenant une face de contact pour coopérer avec une prothèse d'un autre type fixée sur l'autre os de ladite articulation, une face de fixation destinée à être fixée sur ladite surface articulaire de l'os après résection convenable de celui-ci, ladite face de fixation étant munie d'au moins un élément d'ancrage faisant saillie dans ladite face et une surface périphérique reliant lesdites faces de contact et de fixation, chaque élément de prothèse ayant une épaisseur sensiblement constante entre lesdites deux faces,
- un premier ensemble de moyens formant gabarits (136) de contour de résection, chaque gabarit étant associé à une prothèse de l'ensemble, chaque gabarit de contour comportant des moyens de traçage du contour de la résection à réaliser, ledit contour correspondant à la périphérie dudit élément de prothèse;
- un ensemble de moyens formant gabarits de trous d'ancrage (148), chaque moyen formant gabarit étant associé à un des éléments de prothèse de l'ensemble, chaque gabarit de trou d'ancrage comportant des moyens de positionnement par rapport au contour de résection et des moyens de traçage de l'emplacement du ou des trous d'ancrage associés aux éléments d'ancrage ;
un premier ensemble d'instruments de fraisage (140, 140', 142) pour réaliser ladite résection à l'intérieur dudit contour et contrôler les paramètres de fraisage ;
- un deuxième ensemble d'instruments de forage pour réaliser lesdits trous d'ancrage et contrôler les paramètres de forage ; et
- des moyens d'aide au guidage du déplacement desdits instruments de telle manière que les instruments de fraisage soient déplacés au moins selon ledit contour de telle manière que leur axe reste sensiblement parallèle à une direction fixe, ladite direction étant liée auxdits gabarits et que les instruments de perçage soient maintenus avec le même axe de perçage.

7. Procédé de fabrication d'un élément de prothèse d'articulation selon l'une quelconque des revendications 1 à 5, du type dans lequel on part d'un os (120) ayant la forme exacte de la surface articulaire d'un os naturel pour lequel on veut fabriquer la prothèse ;
**caractérisé en ce qu'**il comprend en outre les étapes suivantes :
- on fraise une partie (122, 122') de l'extrémité de l'os selon ladite surface articulaire en maintenant une direction constante de fraisage de manière à définir une zone centrale fraisée et une surface périphérique, la profondeur de fraisage étant sensiblement constante dans ladite zone centrale, ladite surface périphérique définissant au moins en partie un rebord en saillie par rapport à ladite zone centrale selon la direction de fraisage ;
- on réalise un moule (130, 132, 154) dont l'empreinte est limitée d'une part par une première surface définissant la surface de contact, et d'autre part par une deuxième surface constituée par la surface de fixation et ladite surface périphérique résultant du fraisage ;
- on introduit dans ledit moule un matériau de moulage pour que celui-ci prenne la forme de l'empreinte du moule ;
- on démoule la pièce ainsi obtenue par quoi on obtient un prototype dudit élément de prothèse ; et
- on réalise ledit élément de prothèse à partir dudit prototype.

8. Procédé selon la revendication 7, **caractérisé en ce que** ladite première surface est identique à la forme de la surface articulaire de l'os.

9. Procédé selon la revendication 8, **caractérisé en ce que** ladite surface est distincte de la surface articulaire de l'os et est conformée pour réduire les degrés de liberté de l'articulation dont l'os fait partie.

## Claims

1. A joint prosthesis element for fixing to the joint surface of a bone of the joint after said joint surface has been subjected to appropriate resection and for co-operating with at least another prosthesis element fixed to the joint surface of another bone of the joint, said resection defining a projecting rim (22, 98, R, R1) over its outline, said prosthesis element (10, 12, 50, 92) being limited by a contact surface (C, C', 66) for co-operating with the other prosthesis element, said contact surface being substantially identical to the anatomical joint surface, a fixing surface for fixing to the resection of the joint surface, and a peripheral surface (P, P', 38, 74, 76, 102) interconnecting said contact and fixing surfaces, said peripheral surface being designed to co-operate with the projecting rim of said resection, which rim corresponds to the cortical portion of the bone, said fixing surface being provided with fixing means (32, 34, 36, 60, 106, A), said prosthesis element being **characterised in that** said fixing surface (F') is substantially plane, **in that** said contact surface (C') is also substantially plane and substantially parallel to the fixing surface, **in that** said contact surface is directly in contact with the contact surface of the other prosthesis element and **in that** said fixing means are selected from the group comprising adhesive material and at least one anchoring element forming an integral part of said prosthesis, the distance between said contact and fixing surfaces in the central zone thereof and in a fixed direction being substantially constant and greater than 6 mm, said peripheral interconnecting surface flaring relative to said fixed direction from the fixing surface towards the contact surface forming an angle lying in the range 5 to 45 degrees with said fixed direction whereby said peripheral surface is adapted to bear against the rim of said resection.

2. A prosthesis element according to claim 1, **characterised in that** the fixing means comprise a layer of sealing or adhesive material, and **in that** the fixing surface has sufficient roughness to enhance adhesion of said material.

3. A prosthesis element according to claim 1, **characterised in that** the fixing means comprise at least one anchoring element projecting from the fixing surface and forming an integral part of said prosthesis element.

4. A prosthesis element according to any one of claims 1 to 3, **characterised in that** said peripheral surface (P) is substantially in the form of a truncated cone of non-circular right section.

5. A prosthesis element according to claim 3, **characterised in that** the anchoring element (A) comprises at least one peg of substantially frustoconical shape made out of the same material as the remainder of the prosthesis element.

6. A kit for putting a joint prosthesis element according to any one of claims 1 to 5 into place on a joint surface of one of the bones of said joint, the kit being **characterised in that** it comprises:
- a set of similar ones of said prosthesis elements that differ in certain dimensions, each prosthesis element having a contact face for co-operating with a prosthesis of another type fixed on the other bone of said joint, a fixing face for fixing on said joint surface of the bone after it has been subjected to appropriate resection, said fixing face being provided with at least one anchoring element projecting from said face, and a peripheral surface interconnecting said contact and fixing surfaces, each prosthesis element being of substantially constant thickness between said two faces;
- a first set of template-forming means (136) for the outline of the resection, each template being associated with one of the prosthesis of the set, each outline template having means for tracing the outline of the resection to be made, said outline corresponding to the periphery of said prosthesis element;
- a set of template-forming means for the anchoring holes (148), each template-forming means being associated with one of the prosthesis elements of the set, each anchoring hole template including means for positioning relative to the outline of the resection and means for tracing the location of the, or each, anchoring hole associated with the anchoring elements;
- a first set of instruments, for milling (140, 140', 142), for making said resection within said outline and for controlling milling parameters;
- a second set of instruments, for boring, for making said anchoring holes and controlling the boring parameters; and
- means for providing assistance in guiding the displacement of said instruments in such a manner that the milling instruments are displaced at least along said outline in such a manner that their axes remain substantially parallel to a fixed direction, said direction being tied to said templates, and in such a manner that the drilling instruments are held on the same drilling axis.

7. A method of manufacturing a joint prosthesis element according to any one of claims 1 to 5, of the type that starts from a bone (120) having the exact shape of the joint surface of a natural bone for which the prosthesis is to be made;
the method being **characterised in that** it further comprises the following steps:
- milling a portion (122, 122') of the extremity of the bone in said joint surface while maintaining a constant milling direction so as to define a milled central zone and a peripheral surface, the depth of milling being substantially constant in said central zone, said peripheral surface defining at least in part a rim that projects from said central zone in the milling direction;
- making a mould (130, 132, 154) having a mould cavity that is defined firstly by a first surface defining the contact surface and secondly by a second surface constituted by the fixing surface and said peripheral surface that result from the milling;
- putting a moulding material into said mould so as to take the shape of the mould cavity;
- unmoulding the resulting piece, thereby obtaining a prototype for said prosthesis element; and
- making said prosthesis element from said prosthesis.

8. A method according to claim 7, **characterised in that** said first surface is identical in shape to the joint surface of the bone.

9. A method according to claim 8, **characterised in that** said surface is distinct from the joint surface of the bone and is shaped so as to reduce the degrees of freedom of the joint of which the bone forms a part.

## Patentansprüche

1. Gelenkprothesenelement, das dazu bestimmt ist, auf der Gelenkfläche eines Knochens des Gelenks nach entsprechender Resektion der Gelenkfläche befestigt zu werden und mit mindestens einem anderen Prothesenelement, das auf der Gelenkfläche eines anderen Knochens des Gelenks befestigt ist, zusammenzuwirken, wobei die Resektion auf ihrer Kontur einen vorstehenden Wulst (22, 98, R, R1) bildet, wobei das Prothesenelement (10, 12, 50, 92) von einer Kontaktfläche (C, C', 66) abgegrenzt ist, die dazu bestimmt ist, mit dem anderen Prothesenelement zusammenzuwirken, wobei die Kontaktfläche im Wesentlichen mit der anatomischen Gelenkfläche identisch ist, einer Befestigungsfläche, die dazu bestimmt ist, auf der Resektion der Gelenkfläche befestigt zu werden und einer peripheren Fläche (P, P', 38, 74, 76, 102) zum Anschließen der Kontakt- und der Befestigungsflächen, wobei die periphere Fläche dazu bestimmt ist, mit dem vorstehenden Wulst der Resektion, der dem kortikalen Teil des Knochens entspricht, zusammenzuwirken, wobei die Befestigungsfläche mit Befestigungsmitteln (32, 34, 36, 60, 106, A) versehen ist, wobei das Prothesenelement **dadurch gekennzeichnet ist, dass** die Befestigungsfläche (F') im Wesentlichen eben ist, dadurch, dass die Kontaktfläche (C') ebenfalls im Wesentlichen eben und im Wesentlichen parallel zu der Befestigungsfläche ist, dadurch, dass die Kontaktfläche in direktem Kontakt mit der Kontaktfläche des anderen Prothesenelement ist, und dadurch, dass die Befestigungsmittel aus der Gruppe ausgewählt werden, die Vergusswerkstoffe umfasst und mindestens einen Verankerungsdorn, der fester Bestandteil der Prothese ist, wobei die Entfernung nach einer stationären Richtung zwischen der Kontakt- und der Befestigungsfläche in der zentralen Zone im Wesentlichen konstant und größer als 6 mm ist, wobei sich die periphere Anschlussfläche im Vergleich zu der stationären Richtung von der Befestigungsfläche zu der Kontaktfläche aufweitet und mit der stationären Richtung einen Winkel zwischen 5 und 45 Grad bildet, so dass sich die periphere Fläche auf den Wulst der Resektion stützen kann.

2. Prothesenelement nach Anspruch 1, **dadurch gekennzeichnet, dass** die Befestigungsmittel eine Schicht eines Klebe- oder Vergusswerkstoffs enthalten, und dadurch, dass die Befestigungsfläche eine ausreichende Rauheit aufweist, um das Haften des Werkstoffs zu begünstigen.

3. Prothesenelement nach Anspruch 1, **dadurch gekennzeichnet, dass** die Befestigungsmittel mindestens ein Verankerungselement umfassen, das aus der Befestigungsfläche vorsteht und fester Bestandteil des Prothesenelements ist.

4. Prothesenelement nach einem der Ansprüche 1 bis 3, **dadurch gekennzeichnet, dass** die periphere Fläche (P) im Wesentlichen die Form eines Kegelstumpfs mit nicht-kreisförmigem geradem Querschnitt hat.

5. Prothesenelement nach Anspruch 3, **dadurch gekennzeichnet, dass** das Verankerungselement (A) mindestens einen im Wesentlichen kegelstumpfförmigen Dorn umfasst, der mit dem gleichen Werkstoff wie der Rest des Prothesenelements hergestellt ist.

6. Einheit zum Anbringen eines Gelenkprothesenelements nach einem der Ansprüche 1 bis 5 auf einer Gelenkfläche eines Knochens des Gelenks, **dadurch gekennzeichnet, dass** sie Folgendes umfasst:
- eine Einheit der einander ähnlichen Prothesenelemente, die sich durch bestimmte Maße unterscheiden, wobei jedes Prothesenelement eine Kontaktfläche umfasst, um mit einer Prothese eines anderen Typs zusammenzuwirken, die auf dem anderen Knochen des Gelenks befestigt ist, wobei eine Befestigungsfläche dazu bestimmt ist, auf der Gelenkfläche des Knochens nach der entsprechenden Resektion befestigt zu werden, wobei die Befestigungsfläche mindestens mit einem Verankerungselement versehen ist, das in die Fläche vorsteht, und eine periphere Fläche, die die Kontakt- und die Befestigungsfläche verbindet, wobei jedes Prothesenelement zwischen den zwei Flächen eine im Wesentlichen konstante Stärke hat,
- eine erste Einheit Schablonen bildender Mittel (136) mit Resektionskontur, wobei jede Schablone einer Prothese der Einheit zugeordnet ist, wobei jede Konturschablone Mittel zum Ziehen der Kontur der herzustellenden Resektion umfasst, wobei die Kontur der Peripherie des Prothesenelements entspricht,
- eine Einheit von Mitteln, die Schablonen für Verankerungsbohrungen (148) bilden, wobei jedes Schablonenbildungsmittel einem der Prothesenelemente der Einheit zugeordnet ist, wobei jede Verankerungsbohrungsschablone Mittel zum Positionieren zu der Resektionskontur und Mittel zum Ziehen der Stelle der den Verankerungselementen zugeordneten Bohrung oder Bohrungen umfasst,
- eine erste Einheit Fräsinstrumente (140, 140', 142) zum Herstellen der Resektion im Inneren der Kontur und zum Prüfen der Fräsparameter,
- eine zweite Einheit Bohrinstrumente zum Herstellen der Verankerungsbohrungen und zum Prüfen der Bohrparameter und
- Hilfsmittel zum Führen der Bewegung der Instrumente derart, dass die Bohrinstrumente mindestens entlang der Kontur so bewegt werden, dass ihre Achse im Wesentlichen parallel zu einer stationären Richtung bleibt, wobei die Richtung mit den Schablonen verbunden ist, und dass die Bohrinstrumente mit der gleichen Bohrachse gehalten werden.

7. Verfahren zum Herstellen eines Gelenkprothesenelements nach einem der Ansprüche 1 bis 5 des Typs, bei welchem man von einem Knochen (120) ausgeht, der die genaue Form der Gelenkfläche eines natürlichen Knochens hat, für den man die Prothese herstellen will;
**dadurch gekennzeichnet, dass** es ferner die folgenden Schritte umfasst:
- man fräst einen Teil (122, 122') des Endes des Knochens nach der Gelenkfläche, indem man eine konstante Fräsrichtung derart einhält, dass eine zentrale gefräste Zone und eine periphere Fläche definiert werden, wobei die Frästiefe in der zentralen Zone im Wesentlichen konstant ist, wobei die periphere Fläche mindestens zum Teil einen vorstehenden Wulst zu der zentralen Zone nach der Fräsrichtung definiert;
- man stellt eine Form (130, 132, 154) her, deren Abdruck einerseits von einer ersten Fläche abgegrenzt wird, die die Kontaktfläche definiert, und andererseits von einer zweiten Fläche, die aus der Befestigungsfläche und der peripheren Fläche, die sich aus dem Fräsen ergibt, besteht;
- man führt in die Form einen Formwerkstoff ein, damit dieser die Abdruckform der Form annimmt;
- man formt das so erzielte Teil ab, wodurch man einen Prototyp des Prothesenelements erzielt; und
- man stellt das Prothesenelement ausgehend von dem Prototyp her.

8. Verfahren nach Anspruch 7, **dadurch gekennzeichnet, dass** die erste Fläche mit der Form der Gelenkfläche des Knochens identisch ist.

9. Verfahren nach Anspruch 8, **dadurch gekennzeichnet, dass** die Fläche von der Gelenkfläche des Knochens getrennt und ausgebildet ist, um die Freiheitsgrade des Gelenks, zu dem der Knochen gehört, zu verringern.
